# EUROPEAN PATENT APPLICATION

(11) **EP 4 811 276 A1**
(43) Date of publication of application: **23.09.2026**
(21) Application number: 25204498.7
(22) Date of filing: 25.09.2025
(51) Int. Cl.: G06T 7/00

(54) **METHOD AND SYSTEM FOR MADE-TEA QUALITY ASSESSMENT IN MANUFACTURING PROCESS**

(30) Priority: 20.03.2025 IN 202521025312
(71) Applicant: Tata Consultancy Services Limited, Maharashtra (IN)
(72) Inventor: JUNAGADE, Sanket Kailas, 400601 Thane, Maharashtra (IN); BOSE CHOUDHURY, Swagatam, 400601 Thane, Maharashtra (IN); SARANGI, Sanat, 400601 Thane, Maharashtra (IN); SINGH, Dineshkumar Jang Bahadur, 400601 Thane, Maharashtra (IN); PAPPULA, Srinivasu, 500081 Hyderabad, Telangana (IN)
(74) Representative: Goddar, Heinz J.

(57) **Abstract**

This disclosure relates generally to method and system for made-tea quality assessment in manufacturing process. Conventional tea quality assessment methods making it challenging for tea manufacturers to maintain consistent quality standards and meet evolving market demands. The present disclosure collects a physical grade of made-tea sample from the tea factory. Spectral camera captures spectral image of the made-tea sample for preprocessing to obtain improved contrasts. Further, from a plurality of features are extracted from each candidate region to detect a plurality of anomalies, and simultaneously a negative score is assigned based on detected discrepancies. Then, a quality level score is computed to determine a final grade value. The final grade value is assessed based on a quality level score (QLS) and a valuation grade score (VLS). Finally, the method generates a quality assessment report indicating personalized improvements for the tea factory.

## Description

### CROSS-REFERENCE TO RELATED APPLICATIONS AND PRIORITY

The present application claims priority from Indian application no. 202521025312, filed on March 20, 2025.

### TECHNICAL FIELD

The disclosure herein generally relates to tea quality assessment, and, more particularly, to method and system for made-tea quality assessment in manufacturing process.

### BACKGROUND

Tea is recognized as one of the most commonly consumed aromatic beverages globally, renowned for its numerous health benefits. After harvesting, the tea leaves undergo several levels of processing and fermentation to develop their aroma and distinctive flavor. Typically, the processed tea is steeped in water, optionally accompanied by additional ingredients such as milk or sugar, to create what is known as 'made-tea.' However, use of water at a sub-boiling temperature might not eliminate all microbes, including spores of certain bacteria. Although tea has medicinal and antimicrobial principles, it may occasionally contain microbes and contaminated with pathogenic bacteria, yeast and molds during processing and storage, which may pose a potential health risk. As everyday millions of people drink at least one cup of tea, an overall analysis of the contamination profile of different kinds of made tea is of utmost importance and an absolute mandate. Evaluation of various made-tea requires careful consideration of color, smell, and taste of the beverage. There is speculation that the soils used for roadside tea cultivation influence the dynamics of metals from the soil to the tea plants and ultimately to tea infusion, which may be jeopardized by an environment that is becoming more unpredictable and hazardous.

Conventional approaches to assess tea quality, including visual inspection, colorimetry, and sensory evaluation face several challenges. Visual inspection entails analyzing the color, clarity, and general appearance of the tea. However, these approaches are fundamentally subjective and can differ significantly among various observers. For example, Colorimetry employs a colorimeter to quantify tea color offering more objective measurements but lack to consider critical quality attributes. Sensory evaluation depends on a trained panel of tasters to assess aroma, flavor, and overall quality of the tea, which is susceptible to subjectivity. Tasters evaluate tea based on body related to aroma, texture, mouthfeel, and zing associated with taste and physical grade. These approaches utilizes human expertise, which is labor-intensive.

### SUMMARY

Embodiments of the present disclosure present technological improvements as solutions to one or more of the above-mentioned technical problems recognized by the inventors in conventional systems. For example, in one embodiment, a system for made-tea quality assessment in manufacturing process is provided. The system includes randomly collecting a physical grade of a made-tea sample from multiple sections of a production batch in a tea factory, wherein the made-tea sample is formulated with a liquor containing water. Spectral camera under controlled illumination acquire a spectral image of the made-tea sample indicative of chemical composition, wherein the spectral camera captures the spectral image over a predetermined wavelength range to form a multi-dimensional spectral hypercube. Further, each slice of the acquired spectral image is preprocessed by converting each spectral image into grayscale to reduce colour complexity and to improve intensity variations. Then, gaussian blur on each grayscale of corresponding spectral image is applied to smooth random noise and soften sharp edge, and histogram equalization is performed on each blurred image to obtain improved contrast of distinguishable spectral features. Further, spectral segmentation on the pre-processed spectral image is applied to mark one or more regions of interests corresponding to tea particle to create a bounding box on each candidate region among the one or more region of interests. Furthermore, a plurality of features are extracted from each candidate region. Then, the plurality of features are used to detect a plurality of anomalies comprising a spectral anomaly, a visual anomaly and a physical grade anomaly, wherein the spectral anomaly detects deviations with the spectral reflectance data attributable to heavy metal contamination(s), the visual anomaly detects at least one physical contaminants discrepancies, and the physical grade anomaly validates a plurality of morphological features in comparison with factory provided grade standards. Then, a negative score is assigned to each detected anomaly based on severity and type of impact and recommending corrections for each detected anomaly based on the negative score, wherein the negative score includes a high negative indicating critical discrepancies, a moderate negative indicating moderate discrepancies, and a low negative indicating minor discrepancies.

Furthermore, a chemical composition of the made-tea is estimated by correlating the spectral reflectance data with the TDS measurement value, and a quality level score is computed by combining the chemical composition, the physical grade anomaly and the negative score, and categorizing the quality level score into at least one of a high, medium and low based on a predefined threshold. Finally, a final grade value for the made-tea sample is assessed based on the quality level score (QLS) and a valuation grade score (VLS), and the final grade value is compared with a predefined grade threshold to quantify factory standards, wherein the valuation grade score is a quantitative measure of body and zing attributes using the spectral reflectance data and the TDS value. Then, the tea factory is recommended with a quality assessment report indicating personalized improvements based on the final grade value indicative of acceptable quality, the quality level score, and a proximity value, wherein the proximity value is determined based on factory-specific reference spectral profiles.

In another aspect, a method for made-tea quality assessment in manufacturing process is provided. The method includes randomly collecting a physical grade of a made-tea sample from multiple sections of a production batch in a tea factory, wherein the made-tea sample is formulated with a liquor containing water. Spectral camera under controlled illumination acquire a spectral image of the made-tea sample indicative of chemical composition, wherein the spectral camera captures the spectral image over a predetermined wavelength range to form a multi-dimensional spectral hypercube. Further, each slice of the acquired spectral image is preprocessed by converting each spectral image into grayscale to reduce colour complexity and to improve intensity variations. Then, gaussian blur on each grayscale of corresponding spectral image is applied to smooth random noise and soften sharp edge, and histogram equalization is performed on each blurred image to obtain improved contrast of distinguishable spectral features. Further, spectral segmentation on the pre-processed spectral image is applied to mark one or more regions of interests corresponding to tea particle to create a bounding box on each candidate region among the one or more region of interests. Furthermore, a plurality of features are extracted from each candidate region. Then, the plurality of features are used to detect a plurality of anomalies comprising a spectral anomaly, a visual anomaly and a physical grade anomaly, wherein the spectral anomaly detects deviations with the spectral reflectance data attributable to heavy metal contamination(s), the visual anomaly detects at least one physical contaminants discrepancies, and the physical grade anomaly validates a plurality of morphological features in comparison with factory provided grade standards. Then, a negative score is assigned to each detected anomaly based on severity and type of impact, and recommending corrections for each detected anomaly based on the negative score, wherein the negative score includes a high negative indicating critical discrepancies, a moderate negative indicating moderate discrepancies, and a low negative indicating minor discrepancies.

Furthermore, a chemical composition of the made-tea is estimated by correlating the spectral reflectance data with the TDS measurement value, and a quality level score is computed by combining the chemical composition, the physical grade anomaly and the negative score, and categorizing the quality level score into at least one of a high, medium and low based on a predefined threshold. Finally, a final grade value for the made-tea sample is assessed based on the quality level score (QLS) and a valuation grade score (VLS), and the final grade value is compared with a predefined grade threshold to quantify factory standards, wherein the valuation grade score is a quantitative measure of body and zing attributes using the spectral reflectance data and the TDS value. Then, the tea factory is recommended with a quality assessment report indicating personalized improvements based on the final grade value indicative of acceptable quality, the quality level score, and a proximity value, wherein the proximity value is determined based on factory specific reference spectral profiles.

In yet another aspect, there are provided one or more non-transitory machine-readable information storage mediums comprising one or more instructions which when executed by one or more hardware processors cause: randomly collecting a physical grade of a made-tea sample from multiple sections of a production batch in a tea factory, wherein the made-tea sample is formulated with a liquor containing water. Spectral camera under controlled illumination acquires a spectral image of the made-tea sample indicative of chemical composition, wherein the spectral camera captures the spectral image over a predetermined wavelength range to form a multi-dimensional spectral hypercube. Further, each slice of the acquired spectral image is preprocessed by converting each spectral image into grayscale to reduce colour complexity and to improve intensity variations. Then, gaussian blur on each grayscale of corresponding spectral image is applied to smooth random noise and soften sharp edge, and histogram equalization is performed on each blurred image to obtain improved contrast of distinguishable spectral features. Further, spectral segmentation on the pre-processed spectral image is applied to mark one or more regions of interests corresponding to tea particle to create a bounding box on each candidate region among the one or more region of interests. Furthermore, a plurality of features are extracted from each candidate region. Then, the plurality of features are used to detect a plurality of anomalies comprising a spectral anomaly, a visual anomaly and a physical grade anomaly, wherein the spectral anomaly detects deviations with the spectral reflectance data attributable to heavy metal contamination(s), the visual anomaly detects at least one physical contaminants discrepancies, and the physical grade anomaly validates a plurality of morphological features in comparison with factory provided grade standards. Then, a negative score is assigned to each detected anomaly based on severity and type of impact, and recommending corrections for each detected anomaly based on the negative score, wherein the negative score includes a high negative indicating critical discrepancies, a moderate negative indicating moderate discrepancies, and a low negative indicating minor discrepancies.

Furthermore, a chemical composition of the made-tea is estimated by correlating the spectral reflectance data with the TDS measurement value, and a quality level score is computed by combining the chemical composition, the physical grade anomaly and the negative score, and categorizing the quality level score into at least one of a high, medium and low based on a predefined threshold. Finally, a final grade value for the made-tea sample is assessed based on the quality level score (QLS) and a valuation grade score (VLS), and the final grade value is compared with a predefined grade threshold to quantify factory standards, wherein the valuation grade score is a quantitative measure of body and zing attributes using the spectral reflectance data and the TDS value. Then, the tea factory is recommended with a quality assessment report indicating personalized improvements based on the final grade value indicative of acceptable quality, the quality level score, and a proximity value, wherein the proximity value is determined based on factory specific reference spectral profiles.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG.1 illustrates an exemplary system to assess made-tea quality according to some embodiments of the present disclosure.
FIG.2 is a functional block diagram illustrating architecture overview of the system 100 to assess made-tea quality using the system of FIG.1, in accordance with some embodiments of the present disclosure.
FIG.3A and FIG.3B is a flow diagram illustrating a method to assess made-tea quality using the system of FIG.1, in accordance with some embodiments of the present disclosure.
FIG.4 illustrates a spectral segmentation unit to create bounding box using the system of FIG.1, in accordance with some embodiments of the present disclosure.
FIG.5 illustrates a use case example with made-tea structure to assess quality using the system of FIG.1, in accordance with some embodiments of the present disclosure.
FIG.6A illustrates a grayscale spectral image obtained from the preprocessed spectral image, using the system of FIG.1, in accordance with some embodiments of the present disclosure.
FIG.6B illustrates an equalized image of the grayscale spectral image generated by performing histogram equalization using the system of FIG.1, in accordance with some embodiments of the present disclosure.
FIG.6C illustrates a coarse segmented image obtained by applying a clustering mechanism using the system of FIG.1, in accordance with some embodiments of the present disclosure.
FIG.6D illustrates a final segmented image resulted from the coarse segmented image using the system of FIG.1, in accordance with some embodiments of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

### GLOSSARY:

In the context of the present disclosure, the term "made-tea" refers to processed and manufactured form of tea obtained after undergoing various stages of withering, rolling, oxidation, and drying.

The term "spectral segmentation" refers to computational technique used to analyze and classify distinct regions of a spectral image based on wavelengthdependent reflectance characteristics. Spectral segmentation differentiates tea particles from background noise, contaminants, or anomalies by leveraging machine learning and clustering-based methods.

The term "valuation grade score" refers to a quantifiable metric representing the sensory attributes of made-tea, specifically assessing the Body and Zing of the brewed liquor. The valuation grade score is computed by correlating spectral reflectance data with chemical composition features such as polyphenols, theaflavins, caffeine content, and total dissolved solids (TDS). The term valuation grade score is determined using two key parameters: Body and Zing, which are numerically graded and combined into a final two-digit score (VG).

The term "physical grade" refers to categorization of processed tea leaves based on their morphological characteristics, such as particle size, shape uniformity, texture, and overall structural integrity.

The term "factory proximity value" refers to a quantifiable metric representing the similarity or alignment between an analyzed made-tea sample and a factory-specific reference or benchmark sample. The reference samples for proximity analysis may include internally established factory-grade standards, higher valuation tea grades certified by authoritative bodies such as tea boards, or benchmark teas from external sources.

Term "Thearubigins" refers to brown pigment in black tea, are heterogeneous polymers of tea catechins.

Made-tea encompasses different tea grades, including whole leaf, broken leaf, fanning, and dust, which are categorized based on particle size, physical morphology, and quality attributes. Made-tea is the final product before packaging and consumption, which can be brewed in hot water to extract its flavors, aroma, and chemical compounds. The present disclosure addresses conventional tea grading challenges which is predominantly based on human sensory evaluation, which can be influenced by personal preferences, individual experience, and various environmental conditions. This inherent subjectivity may result in inconsistencies and disputes in grading, thereby affecting quality control and transparency. In contrast, spectral analysis offers objective, quantitative data derived from the tea's spectral signature, thereby removing the human factor and its associated biases. Furthermore, conventional techniques such as visual assessment, colorimetry, and sensory evaluation can be labor-intensive, particularly when handling large volumes of tea. Spectral analysis can be executed for simultaneous analysis of multiple samples and, in some systems, providing real-time data. This capability significantly diminishes the time needed for quality control, enhancing efficiency and throughput. Additionally, traditional methods can be destructive, necessitating physical or chemical modifications to the sample. They frequently involve the use of hazardous chemicals and produce waste, which poses environmental concerns. Consequently, conventional methods are often impractical for large-scale analysis due to their time limitations. Therefore, the present disclosure intends to review the level of microbial load associated with made-tea to generate report accumulated indicative of the presence of different bacterial pathogens in tea leaves, besides its contamination by pathogenic fungal strains and mycotoxins for factory improvements.

Embodiments of the present disclosure herein provides method and system for assessing quality of made-tea. The system 100 obtains physical grade of made-tea sample(s) from multiple sections of a production batch in a tea factory to assess quality. The made-tea sample further undergoes several stages such as detection, analysis, grading, and quality prediction for assessment of made-tea quality. The method of the present disclosure integrates custom deep learning based segmentation models with clustering techniques to analyze at least one morphological feature and at least one spectral feature from the made-tea sample(s). Furthermore, spectral camera parameter imaging analysis is combined with an anomaly detection framework which ensures optimal imaging conditions enables accurate detection and in-depth analysis of made-tea sample characteristics, addressing challenges of existing techniques. Moreover, the method of the present disclosure incorporates efficient grading and quality prediction phase that merges spectral reflectance data with Total Dissolved Solids (TDS) measurements. This integration aids in forecasting essential chemical properties and establishing a grading system that encompasses chemical composition, physical characteristics, and sensory attributes. The system features a scoring mechanism and machine learning-based valuation grading which provides comprehensive assessments of made-tea quality. By leveraging various data sources and specialized analytical models, the system delivers an holistic assessment of made-tea quality through an integrated methodology. Additionally, the method emphasizes provision of personalized recommendations for tea production, blending, and brewing through the use of convolutional neural networks (CNN) and quality gap analysis.

The system 100 forecasts brewing quality attributes and identifies gaps between existing and desired quality profiles. Based on these forecasts, it formulates recommendations for optimizing brewing parameters and manufacturing processes. Furthermore, the method employs analytical techniques to ensure consistent blending by managing blending ratios and uniformity. These personalized, data-driven recommendations enhance both consumer experience and production efficiency, offering a solution that uniquely combines quality assessment with optimization strategies. The method offers significant advantages in delivering comprehensive and precise quality assessments, as well as tailored production strategies.

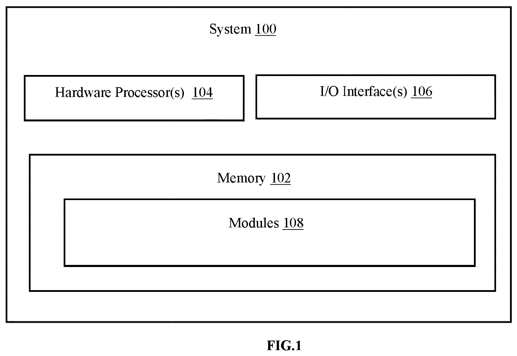

Referring now to the drawings, and more particularly to FIG.1 through FIG.6D, where similar reference characters denote corresponding features consistently throughout the figures, there are shown preferred embodiments and these embodiments are described in the context of the following exemplary system and/or method.

FIG.1 illustrates an exemplary system to assess made-tea quality according to some embodiments of the present disclosure. In an embodiment, the system 100 includes a processor(s) 104, communication interface device(s), alternatively referred as input/output (I/O) interface(s) 106, and one or more data storage devices or a memory 102 operatively coupled to the processor(s) 104. The system 100 with one or more hardware processors is configured to execute functions of one or more functional blocks of the system 100.

Referring to the components of system 100, in an embodiment, the processor(s) 104, can be one or more hardware processors 104. In an embodiment, the one or more hardware processors 104 can be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in the memory 102. In an embodiment, the system 100 can be implemented in a variety of computing systems including laptop computers, notebooks, hand-held devices such as mobile phones, workstations, mainframe computers, servers, and the like.

The I/O interface(s) 106 may include a variety of software and hardware interfaces, for example, a preprocessing unit 202, a spectral segmentation unit 204, a feature extractor 206, an anomaly detection unit 208, a chemical composition unit 210, and the like are provided that facilitate multiple communications within a wide variety of networks N/W and protocol types, including wired networks, for example, LAN, cable, etc., and wireless networks, such as WLAN, cellular and the like. In an embodiment, the I/O interface (s) 106 can include one or more ports for connecting to a number of external devices or to another server or devices. The system 100 receives inputs from user via an application installed on user-end devices connected to the system 100 such as a laptop, handheld device or the like.

The memory 102 may include any computer-readable medium known in the art including, for example, volatile memory, such as static random access memory (SRAM) and dynamic random access memory (DRAM), and/or non-volatile memory, disks, optical disks, and magnetic tapes.

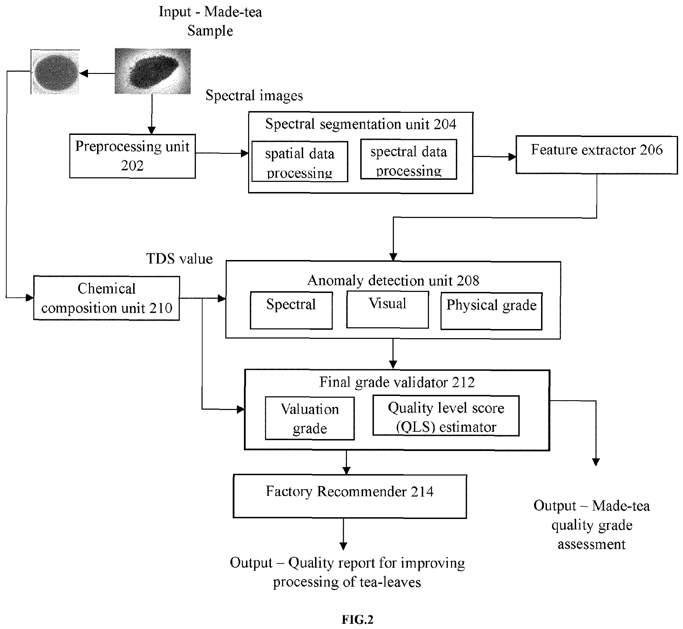

In an embodiment, the memory 102 includes a plurality of modules 110 such as an preprocessing unit 202, a spectral segmentation unit 204, a feature extractor 206, an anomaly detection unit 208, a chemical composition unit 210 and so on as depicted in FIG.2. The plurality of modules 110 include programs or coded instructions that supplement applications or functions performed by the system 100 for made-tea quality assessment being performed by the system 100. The plurality of modules 110, amongst other things, can include routines, programs, objects, components, and data structures, which performs particular tasks or implement particular abstract data types. The plurality of modules 110 may also be used as, signal processor(s), node machine(s), logic circuitries, and/or any other device or component that manipulates signals based on operational instructions. Further, the plurality of modules 110 can be used by hardware, by computer-readable instructions executed by the one or more hardware processors 104, or by a combination thereof. The plurality of modules 110 can include various sub-modules (as shown in FIG.2).

Further, the memory 102 may comprise information pertaining to input(s)/output(s) of each step performed by the processor(s) 104 of the system 100 and methods of the present disclosure. Further, the memory 102 includes a database 108. Although the database 108 is shown internal to the system 100, it will be noted that, in alternate embodiments, the database 108 can also be implemented external to the system 100, and communicatively coupled to the system 100. The data contained within such external database may be periodically updated. For example, anomalies samples may be added into the database (not shown in FIG.1) and/or existing data may be modified and/or non-useful data may be deleted from the database. In one example, the data may be stored in an external system, such as a Lightweight Directory Access Protocol (LDAP) directory and a Relational Database Management System (RDBMS).

FIG.2 is a functional block diagram illustrating architecture overview of the system 100 to assess made-tea quality using the system of FIG.1, in accordance with some embodiments of the present disclosure.

In a tea manufacturing facility, it is crucial to achieve improvements and deliver accurate recommendations for adjusting processing parameters during stages such as oxidation and drying. Combined integration of image processing techniques, spectral analysis, machine learning algorithms, and factory-specific recommendations guarantees thorough quality evaluation. The proposed methodology uniquely combines spectral imaging data, which reveals insights into chemical composition with spatial characteristics of foreign objects. This integration allows a comprehensive evaluation of made-tea quality by examining both visual and chemical irregularities within a unified framework. The proposed method assists in identifying made-tea quality variations between currently produced tea samples and the desired target profiles, thereby highlighting specific areas for enhancement. This enables identification of both visual and spectral irregularities, calculates quality scores, and provides actionable insights to refine manufacturing processes. This seamless integration of these elements ensures accurate and dependable quality assessments, aligning production results with established quality benchmarks.

In one embodiment, the method of the present disclosure provides an unified assessment framework to improve made-tea quality in tea manufacturing factory. The system 100 is deployed at the tea manufacturing factory integrated with spectral camera to perform quality assessment. The system 100 includes the preprocessing unit 202, the spectral segmentation unit 204, the feature extractor 206, the anomaly detection unit 208, the chemical composition unit 210, the final grade validator 212, and the factory recommender 214.

Initially, freshly made-tea samples are processed using standard factory methods for uniformity and stored under controlled conditions to maintain freshness and prevent degradation. These samples are received by the preprocessing unit 202 from the production batch of the tea manufacturing factory. The spectral camera deployed in the image tea manufacturing factory captures spectral images under controlled light illumination. The captured spectral images are preprocessed for high resolution eliminating blurred image. Further, the spectral segmentation unit 204 applies spectral segmentation on the pre-processed spectral image to crop region of interest and creates a bounding box on each candidate region. Also, quantitative measure of dissolving solids in liquor with water is identified by the chemical composition unit 210. Herein, liquor can be understood as mixture of made-tea sample and water.

Then, the feature extractor 206 extracts a plurality of features from each candidate region to extract relevant parameters associated with each feature. Each extracted feature is optimized with relevant spectral and morphological features from the segmented samples, enabling accurate quality assessment and anomaly detection.

Further, the anomaly detection unit 208 detects a plurality of anomalies associated with each feature to ensure product purity and for quality standard adherence. Each anomaly is provided a weightage to indicate severity based on ranks.

Also, quantitative measure of dissolving solids in liquor with water is identified by the chemical composition unit 210. Here, liquor being mixture of made-tea sample and water. This estimates concentrations of key chemical compounds (e.g., caffeine, theaflavins) using spectral reflectance data and TDS measurements.

Then, the quality level score estimator 212 estimates the quality level score using the output of chemical composition unit 210 and the anomaly detection unit 208 and the plurality of features indicating threshold based quality prediction. Finally, the final grade validator 214 validates predicts valuation grade of the made-tea sample using body and zing parameters. The quality level score estimator 212 provides replicate of the evaluations typically conducted by multiple tea tasters. This ensures objective, consistent, and efficient grading that aligns with industry standards, while eliminating the time and resource demands of brewing samples for assessment.

Finally, the factory recommender 214 provides final tea grade insights with an quality assessment report using the outputs of the quality level score estimator 212 and the final grade validator 214 and a proximity value. The quality assessment report provides insights comparing current made-tea samples to match a predefined target quality profiles and suggest adjustments to the tea manufacturing facility. The proximity value is determined based on factory specific reference spectral profiles determined based on current trends and market needs.

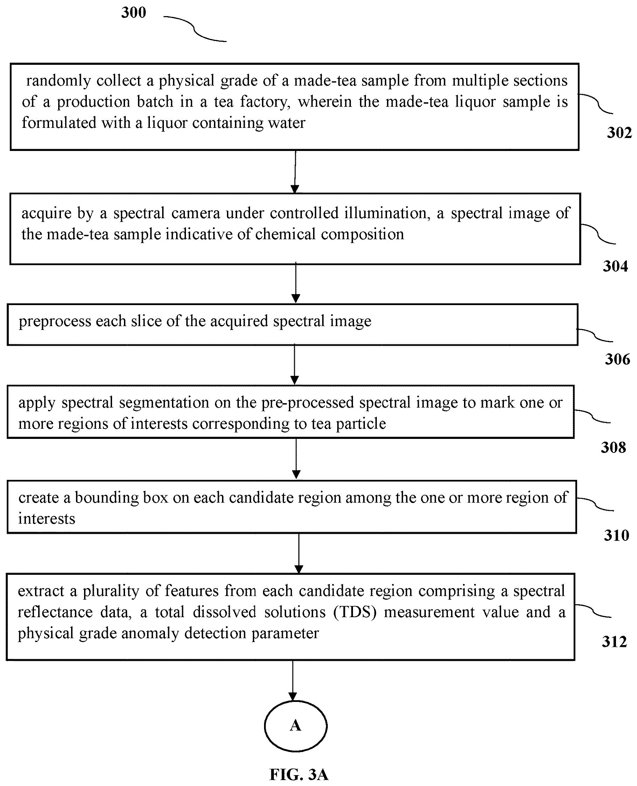

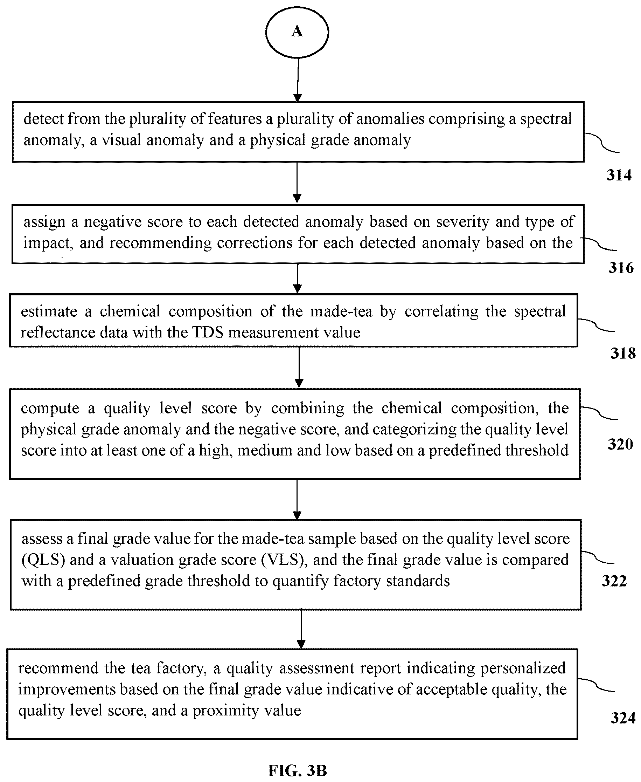

FIG.3A and FIG.3B illustrating a method 300 to assess made-tea quality using the system of FIG.1, in accordance with some embodiments of the present disclosure. In an embodiment, the system 100 comprises one or more data storage devices or the memory 102 operatively coupled to the processor(s) 104 and is configured to store instructions for execution of steps of the method 300 of the present disclosure will now be explained with reference to the components or blocks of the system 100 as depicted in FIG.2 through FIG.6D in accordance with an example embodiment of the present disclosure.

Although process steps, method steps, techniques or the like may be described in a sequential order, such processes, methods, and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

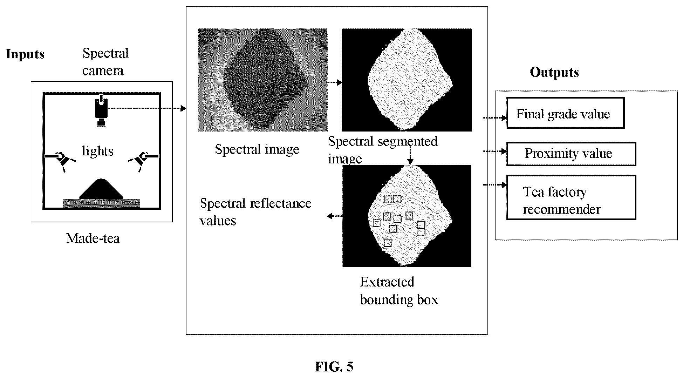

Referring to FIG.3A and FIG.3B and the steps of the method 300, at step 302 of the method 300, the one or more hardware processors 104 of the system 100 are configured by the instructions to randomly collect physical grade of a made-tea sample from multiple sections of a production batch in a tea factory, wherein the made-tea liquor sample is formulated with a liquor containing water. Considering an example (referring FIG.5), freshly made-tea samples representative of current production batch are obtained from the tea factory or the manufacturing facility as inputs for quality assessments. Made-tea sample(s) are randomly selected from a physical grade of different sections of the production batch having different physical grade(s) to capture variability. This ensures made-tea samples are free from visible defects or contaminants. The made-tea sample is prepared by combining processed tea leaves with liquor samples. The liquor sample may be, for example water used to brew the made-tea sample for spectral imaging.

The preparation of made-tea samples involves evenly distributing the freshly made tea on a standardized substrate such as white parchment paper, while minimizing overlaps to ensure clear visibility. Additionally, optimal arrangement is achieved by positioning the made-tea samples to uniformly cover the imaging area, thereby preventing edge effects. It is advisable to utilize fixtures or molds to ensure consistent thickness across the samples. For sample storage and handling, it is essential to maintain a controlled environment by storing the prepared samples at regulated temperature and humidity levels to prevent moisture fluctuations. To minimize the risk of contamination, made-tea samples should be handled using clean, sterilized tools to avoid any cross-contamination. Regarding the brewing parameters for made-tea liquor samples, they are typically brewed with water at a lower temperature of approximately 85°C and steeped for a brief duration of 2.5 minutes. This brewing method accentuates the delicate and floral flavor profile of the tea. The output consists of made-tea samples that are uniformly distributed, appropriately stored, and prepared for high-resolution spectral imaging and subsequent quality evaluation.

At step 304 of the method 300, the one or more hardware processors 104 is configured to acquire by a spectral camera under controlled illumination, a spectral image of the made-tea sample indicative of chemical composition, wherein the spectral camera captures the spectral image over a predetermined wavelength range to form a multi-dimensional spectral hypercube.

The preprocessing unit 202 of the system 100 obtains the spectral image of the made-tea sample captured by the spectral camera wit range from 400-2500nm under controlled lighting setup with consistent illumination. Each spectral image may be saved as .raw, .hdr, and .jpeg formats.

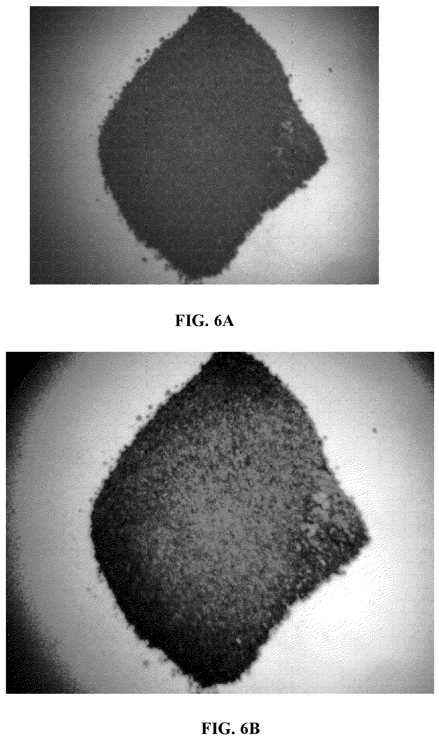

At step 306 of the method 300, the one or more hardware processors 104 is configured to preprocess each slice of the acquired spectral image. In an embodiment, the preprocessing unit 202 processes each spectral image to convert into grayscale to reduce colour complexity and to improve intensity variations. Referring to FIG.6A, grayscale reduces computational complexity. Gaussian blur is applied on each grayscale of corresponding spectral image to smooth out random noise and soften sharp edge. Then, histogram equalization (FIG.6B) is applied on each blurred image to improve contrast of distinguishable spectral features.

At step 308 of the method 300, the one or more hardware processors 104 is configured to apply spectral segmentation on the pre-processed spectral image to mark one or more regions of interests corresponding to tea particle.

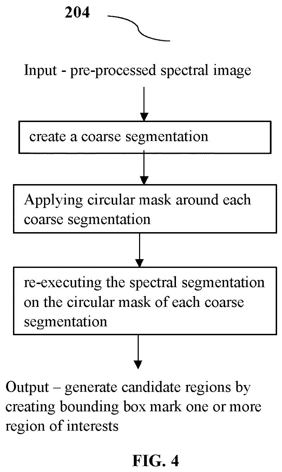

For the output received from the preprocessing unit 202, referring now FIG.4, the spectral segmentation unit 204 applies multi-layered spectral segmentation on the pre-processed spectral image to identify and refine regions of interest (ROI), ensuring robust spectral differentiation. This process involves applying at least one clustering mechanism (e.g., K-means or Gaussian Mixture Models (GMM)) to partition the spectral image into clusters based on spectral reflectance variations. This step creates an initial candidate region map differentiating tea particles from background and outlier pixels. Here, the clustering mechanism may be for example K-means or GMM to segment the image by spectral reflectance. As known in the art K-means clustering is an unsupervised learning algorithm partitions the spectral image into K-distinct clusters by minimizing variance within each cluster. It iteratively assigns each pixel to the nearest cluster centroid based on spectral reflectance values, recalculates centroids, and converges when the cluster assignments stabilize. K-means is computationally efficient and well-suited for segmenting tea particles based on dominant spectral characteristics.

Gaussian Mixture Models (GMM) extend the segmentation approach by assuming that the spectral reflectance distribution of made-tea samples follows a mixture of multiple Gaussian distributions. Unlike K-means, which assigns hard cluster memberships, GMM provides a probabilistic assignment where each pixel has a likelihood of belonging to multiple clusters. This allows better handling of overlapping spectral signatures in complex tea blends where multiple grades may share similar reflectance profiles but exhibit subtle variations in intensity or spectral absorption.

By leveraging these clustering techniques, the spectral segmentation process ensures an adaptive and data-driven classification of tea particles, enabling robust spectral differentiation while reducing background noise and outliers that may otherwise affect the accuracy of subsequent feature extraction and anomaly detection stages.

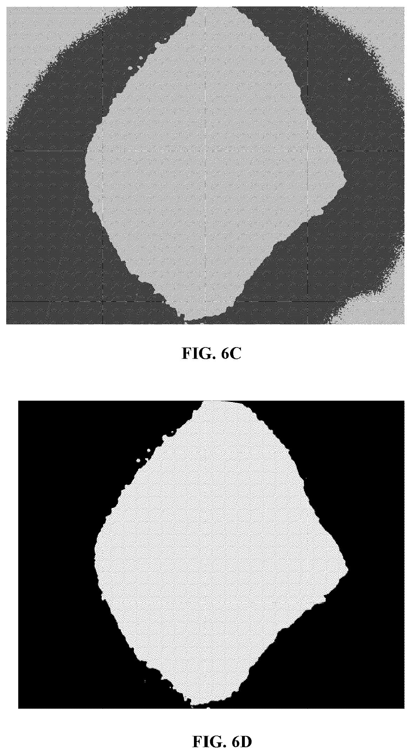

Further, a circular mask is created around each coarse segmentation (referring now FIG.6C) of the pre-processed spectral image. The circular mask is dynamically determined based on a minimal radius which is computed using at least one of a first radius and a second radius. The first radius is set to half of the smallest image dimension excluding peripheral noise to prevent false segmentation due to edge artifacts. The second radius is determined based on the edge of the noisy boundary identified after the initial clustering-based segmentation, ensuring that regions affected by background noise or unwanted reflections that are excluded from subsequent analysis.

Referring now FIG.6D, upon defining the circular mask, the spectral segmentation process is re-executed within the masked region. The spectral segmentation technique excludes edges and noise-affected regions thereby refining the candidate regions corresponding to valid made-tea particles. Also, the segmentation step isolates homogeneous spectral regions within the circular mask ensuring that the bounding box placement is optimized for accurate spectral and morphological analysis. Further, a hierarchical segmentation refinement is performed wherein an adaptive thresholding mechanism dynamically filters out false-positive segmentations, allowing only the most relevant spectral regions to be passed for bounding box creation. The final segmented candidate regions serve as the primary input for bounding box generation, ensuring that bounding boxes are created over highly relevant regions while minimizing redundant data inclusion. By incorporating circular masking and hierarchical segmentation refinement, the system ensures that bounding boxes are precisely placed over high-integrity spectral data, thereby enhancing the accuracy of subsequent tea quality assessment models.

At step 310 of the method 300, the one or more hardware processors (104) are configured to create a bounding box on each candidate region among the one or more regions of interest, wherein the bounding box serves as a localized area for spectral and morphological feature extraction within the made-tea sample.

The spectral segmentation unit 204 creates a dynamic non-overlapping bounding box on each candidate region by scanning a masked version of the preprocessed spectral image to identify one or more non-zero pixel clusters, wherein the non-zero clusters correspond to valid made-tea regions of interest. Bounding box selection technique ensures that each bounding box lies within the image boundaries of the preprocessed spectral image. The bounding box does not overlap with previously established bounding boxes or non-segmented regions. The bounding box encloses the localized region of the made-tea particle while excluding extraneous background pixels or noise. An aspect ratio constraint is applied to prevent excessively elongated or distorted bounding boxes, ensuring optimal spectral reflectance extraction.

The bounding box selection follows custom non-overlapping bounding box technique which scans the masked spectral image to detect segmented non-zero clusters. Further, a starting point is selected randomly within the non-zero pixel clusters to minimize systematic bias. Then, each bounding box is validated by checking overlap function that ensures that new bounding boxes do not overlap existing ones. Then, region of interest (ROI) are extracted for each bounding box while discarding invalid or noisy regions. This rectangles overlap function prevents merging of bounding boxes with adjacent segmented areas. Then, the bounding box placement is refined by iteratively adjusting dimensions, location, and pixel density until an optimal non-overlapping set of bounding boxes is created. The top-left corner coordinates of the bounding box are added to the coordinates list, enabling spatial indexing for feature extraction. Then, a counter mechanism tracks the number of successfully added bounding boxes, ensuring that the required number of bounding boxes is generated for optimal feature extraction. The method tracks bounding box properties (width, height, coordinates, aspect ratio) to ensure uniform spatial indexing for feature extraction. To enhance computational efficiency, Principal Component Analysis (PCA) is applied, reducing redundant feature variations while retaining high-variance components.

To enhance computational efficiency, a non-overlapping bounding box selection algorithm is executed, wherein a secondary validation step ensures bounding box regions do not merge with adjacent segmented areas, preventing contamination of spectral data. Bounding box density is optimized, ensuring uniform distribution across the sample to capture variations in the made-tea quality effectively.

The bounding box for each candidate region is created by performing bounding box verification based on at least one of the criteria: (a) each candidate region lies within boundaries of the preprocessed spectral image masks, (b) each candidate region must not overlap with previously defined bounding boxes or non-segmented regions, (c) each candidate region covers localized regions of the made-tea tea particle without enclosing extraneous pixels and (d) each candidate region boundary box regions optimal aspect ratio constraints are constant.

At step 312 of the method 300, the one or more hardware processors 104 is configured to extract a plurality of features from each candidate region, wherein the plurality of features includes (i) a spectral reflectance data indicative of chemical profile from the bounding box region superimposed on raw spectral hypercube, (ii) a total dissolved solutions (TDS) measurement value obtained from the liquor used to prepare made-tea sample indicative of approximate dissolved compounds, and (iii) a physical grade anomaly detection parameter identifying presence of foreign particles.

Once each candidate region is obtained from the above step 310, the plurality of features are extracted by the feature extractor 206. The plurality of features includes the spectral reflectance data, the total dissolved solutions (TDS) measurement value and the physical grade value.

The spectral reflectance data is obtained by performing bounding box analysis with average reflectance intensity across wavelengths in bounding boxes. Spectral reflectance calibration is performed to superimpose pixel locations on raw data to normalize imaging inconsistencies which is indicative of chemical profile.

The Total Dissolved Solids (TDS) measurement value of the liquor is prepared by brewing the made-tea sample under standardized conditions. Then, TDS calculation is a measure of dissolved solids with a TDS meter for chemical composition prediction.

Now at step 314 of the method 300, the one or more hardware processors 104 is configured to detect from the plurality of features, a plurality of anomalies comprising a spectral anomaly, a visual anomaly and a physical grade anomaly, wherein the spectral anomaly detects deviations in the spectral reflectance data attributable to heavy metal contamination(s), the visual anomaly detects physical contaminants discrepancies, and the physical grade anomaly validates morphological features in comparison with factory provided grade standards.

Referring to the above example, the anomaly detection unit 208 identifies and quantifies anomalies present within the made-tea samples to ensure quality, purity, and standard adherence. The plurality of features detects the plurality of anomalies associated with the made-tea sample. For example, the spectral anomaly among the plurality of anomalies are flagged for deviations from the spectral reflectance data attributable to either heavy metal contamination or aberrant camera parameters.

The spectral anomaly is detected from continuously monitoring the parameters of the spectral camera, including gain, exposure time, brightness, contrast, and shutter speed. Adjustments to these parameters are made dynamically, but only when the detected anomaly is linked to discrepancies associated with the spectral camera, in order to enhance the calibration of spectral reflectance. If spectral inconsistencies continue after making camera adjustments, the intensity of the light source is altered to maintain consistent reflectance conditions. Conversely, if the anomaly is attributed to heavy metal contamination, no modifications are made to the camera parameters or the illumination, as these anomalies are fundamentally chemical and necessitate a distinct contamination analysis. Finally, the spectral reflectance data from each bounding box is compared against a reference spectral reflectance dataset to identify deviations, quantify the contaminants present, and evaluate the extent of variation.

For example, to detect the spectral anomaly, Support Vector Machines (SVM), Random Forest (RF), or similar classification models are used to classify spectral reflectance deviations. SVM is applied to high-dimensional spectral feature space to distinguish between normal and contaminated tea particle spectra, where a hyperplane separates contaminated and uncontaminated spectral regions based on training data. Alternatively, Random Forest classifiers leverage multiple decision trees to assess spectral deviations caused by heavy metal contamination, ensuring robustness against spectral variations. The model accounts for dynamically calibrated spectral reflectance values by incorporating real-time camera adjustments (gain, brightness, contrast, shutter speed) and illumination intensity modifications to minimize false positives.

The spectral anomalies may include for example, heavy metal contamination such as iron, lead, cadmium, or mercury, residual pesticide traces, or significant spectral distortions caused by suboptimal camera parameters or improper illumination intensity, which affect spectral integrity.

The visual anomaly is detected from the plurality of features to identify physical contaminants discrepancies present in the made tea by detecting physical contaminants such as foreign objects within the made-tea samples using image processing techniques. Multi-spectral data captured at different wavelength intervals are compared to enhance differentiation between organic made-tea structures and non-organic contaminants. A dynamically calibrated reflectance threshold is applied to improve detection accuracy, ensuring that variations due to camera setting changes do not lead to false anomaly detection.

For example, a Convolutional Neural Network (CNN)-based object detection model is used to detect objects and identify physical contaminants with high precision and recall. The CNN model processes images captured in either a three-band or a single-band grayscale format, depending on spectral imaging capabilities. The three-band CNN model utilizes multi-wavelength spectral input (e.g., visible + infrared + near-infrared bands) to enhance material differentiation, while the one-band CNN model focuses on grayscale textures to highlight anomalies in monochromatic spectral data.

The visual anomalies may include paper, plastic, fabric fibres, wood fragments, and other non-tea contaminants that do not conform to standard made-tea characteristics. Additionally, the model is trained to detect shredded materials, such as unwanted thread particles or small packaging residues, which can be mistakenly introduced during processing.

The physical grade anomaly is detected from the processed tea leaves used to prepare made-tea by extracting a plurality of morphological features from each bounding box and comparing them with a predetermined physical grade benchmark. The extracted features include shape descriptors, particle size distribution, edge sharpness, and surface texture uniformity. Finally, a pattern recognition technique is applied to validate the detected morphological features against known factory-defined standards, and an alert is triggered when substantial deviations suggest adulteration or mislabeling.

For example, the pattern recognition technique may involve clustering-based classification (e.g., K-means, Gaussian Mixture Models (GMM)) or supervised machine-learning models, including Support Vector Machines (SVM), Random Forest (RF), and Gradient Boosting Decision Trees (GBDT), as well as deep-learning architectures such as Convolutional Neural Networks (CNNs) and Vision Transformers (ViTs) with spectral attenuation layer. These techniques are used to automate physical grade validation by correlating extracted morphological features with pre-classified tea grade distributions, ensuring high accuracy in physical grade verification and anomaly detection.

At step 316 of the method 300, the one or more hardware processors 104 is configured to assign a customizable negative score to each detected anomaly based on severity and type of impact, and recommend corrections for each anomaly based on the negative score, wherein the negative score includes a high negative score for critical discrepancies, a moderate negative score for moderate discrepancies, and a low negative score for minor discrepancies. The negative scoring parameters are dynamically adjustable based on factory-specific quality standards and can be finetuned according to production line requirements.

Once the plurality of anomalies are detected in step 314, each anomaly is assigned with a customizable negative score based on severity and the impact type. The scoring parameters are dynamically adjusted based on factory-specific quality standards, ensuring that scoring thresholds adapt based on real-time production data and historical trends. The negative score is defined in Equation 1, $negativescore = \left(\frac{{\sum }_{i = 1}^{n} {S}_{i}}{T}\right) ∗ 100$ Where, *Sᵢ* is defined based on anomaly parameters, *T* is defined as total assigned score for each made-tea sample. If certain anomalies (e.g., heavy metal contamination) are consistently detected across batches, the system prompts user intervention to review and confirm adjustments before dynamically modifying the anomaly severity scores to reflect cumulative factory-specific risks. For example, the negative scoring may be as defined in Table 1,

**Table 1 - Negative Scoring for anomaly parameters**

| | |
|---|---|
| Spectral camera parameter anomalies : | |
| | Gain deviation: -50 points |
| | Shutter Speed (Exposure time) deviation: -40 points |
| | Brightness deviation: -30 points |
| | Contrast deviation: -30 points |
| | Illumination intensity adjustment required: -20 points |
| Heavy metal contamination: | |
| | Iron contamination: -25 points |
| | Other heavy metals: -30 points |
| Visual anomalies | |
| | Paper: -10 points |
| | Thread: -10 points |
| | Plastic: -15 points |
| | Wood: -15 points |
| Other visual contaminants: -10 points, and customization scores may be factory specific. | |

The high negative score is assigned to detected spectral anomalies if camera-related parameters are identified out of tolerance, signifying an elevated risk of misinterpretation of spectral data and the need for immediate recalibration. If multiple interdependent anomalies (e.g., both spectral and morphological irregularities) are detected in the same sample, an adaptive weighting function prioritizes anomalies with a greater impact on tea quality. Additionally, if spectral deviations align with unusual morphological characteristics, such as irregularities in particle shape or size distribution, the anomaly is further emphasized as a combined spectral-morphological inconsistency, which demands both spectral adjustment and morphological verification.

The low negative score is assigned to visual anomalies such as foreign contaminants, if they reflect relatively lower impact compared to camera and spectral anomalies. However, if a visual anomaly is correlated with spectral inconsistencies (e.g., an unidentified material with unusual spectral reflectance), the system applies a fused spectral-morphological validation check, adjusting the anomaly score dynamically. This prevents underestimating contaminants that could have a greater effect on quality but were initially flagged with a lower severity.

The moderate negative score is assigned to physical grade anomalies, particularly if harmful metal contaminants are detected. The severity score is dynamically adjusted based on the concentration levels of contaminants detected, ensuring that minor deviations do not receive the same penalty as critical chemical irregularities. This ensures that misclassified grades or adulterated samples are flagged appropriately based on combined chemical and structural irregularities rather than a fixed scoring threshold.

Now at step 318 of the method 300, the one or more hardware processors (104) is configured to estimate the chemical composition of made-tea by correlating the spectral reflectance data with the TDS measurement value, ensuring a quantitative assessment of chemical compounds present in the made-tea sample for quality verification.

Here, the chemical composition unit (210) estimates the chemical composition of key tea compounds, including caffeine, theaflavins, catechins, polyphenols, and flavonoids, by analyzing spectral reflectance data and TDS measurement values. This applies a spectral fingerprinting approach where specific wavelength absorption patterns are mapped to known chemical concentrations to achieve precise estimation.

Data correlation models used for chemical composition estimation may include Support Vector Regression (SVR), Deep Neural Networks (DNNs, CNNs, LSTMs), Random Forest (RF), Gradient Boosting Decision Trees (GBDT), Bayesian Regression models, among others. The referred data correlation models are trained using datasets containing known compound concentrations, spectral reflectance curves, and TDS values.

For example, known compound concentrations may be determined through standardized manual chemical tests, including wet chemistry methods such as titration for poly-phenol estimation, Kjeldahl analysis for nitrogen-based compound quantification, gravimetric methods for moisture assessment, Soxhlet extraction for caffeine determination, and a TDS meter for measuring total dissolved solids (TDS) in brewed tea liquor. These manual chemical tests establishes ground truth values are used to generate labeled datasets for training machine learning models. The acquired chemical composition data is correlated with corresponding spectral reflectance patterns ensuring the models learn accurate associations between chemical properties and imaging features. This method enhances the reliability of chemical composition estimation by integrating empirical lab-tested results with computational analysis, thereby improving the robustness and accuracy of predictive models.

These parameters are optimized using hyper-parameter tuning, feature selection, and cross-validation techniques, such as k-fold cross-validation, Bayesian optimization, and grid search tuning, to ensure robustness and model generalization.

Further, at step 320 of the method 300, the one or more hardware processors (104) is configured to compute a quality level score (QLS) by combining the chemical composition score, the physical grade anomaly score, and the negative score, and categorizing the quality level score into at least one of high, medium, or low, based on a predefined threshold. The quality level score is categorized based on the predefined thresholds such as for high quality if QLS ≥ 0.80, medium quality if 0.60 ≤ QLS < 0.80, and low quality if QLS < 0.60.

The final grade validator 212 computes the quality level score (QLS) based on the chemical composition score (CS), the physical grade average score (PGAS), and a weighting factor (W1, W2). The chemical composition score (CS) is calculated as the difference between the measured concentration of a compound and its optimal concentration, normalized across all chemical compounds, as shown in Equation 2, $CS = \frac{1}{n} {\sum }_{i = 1}^{n} \left(1-\frac{\left|{c}_{i}-{c}_{opt , i}\right|}{{c}_{opt , i}}\right)$

Where, *cᵢ* is Measured concentration of compound i. *c_{opt,i}* is Optimal concentration of compound i.n is total number of chemical compounds analysed. The quality level score is defined below in Equation 3, $qualitylevelscore = {w}_{1} ∗ CS + {w}_{2} ∗ PGAS$

Where, *w*₁ and *w*₂ are weighting factors such that *w*₁+*w*₂=l.

The physical grade average score (PGAS) is calculated by multiplying the proportion of each physical grade in the sample with its predefined physical grade score, as shown in Equation 4: $PGAS = {\sum }_{j = 1}^{m} {p}_{j} ∗ {PGS}_{j}$

Where, m is number of different physical grades in the sample, *pⱼ* is proportion of physical grade j in the sample defined between 0 to 1 such that ${\sum }_{j = 1}^{m} {p}_{j} = 1$*.*

For example broken orange pekoe (BOP) is 70% and broken orange pekoe small tea (BOPSM) as 30%. then it will be 0.7 and 0.3 respectively. *PGSⱼ* is predefined physical grade score assigned to grade j based on industry standards, expert evaluation, or user-defined grading systems.

The predefined physical grades of Black Tea Score (PGS) for different types of black tea are listed in below Table 2. These values serve as default references for quality assessment in black tea processing. However, PGS values can be modified or defined separately for other made-tea varieties, including green tea, oolong tea, white tea, and fermented teas, based on factory-specific quality assessment criteria as in Table 1,

**Table 2 - Physical grades of Black Tea Score (PGS)**

| Category | Physical Grade | PGS (Physical Grade Score) |
|---|---|---|
| Whole Leaf | FTGFOP | 1.0 |
| | TGFOP | 0.95 |
| | GFOP | 0.90 |
| | FOP | 0.85 |
| | OP | 0.80 |
| Broken Leaf | FBOP | 0.75 |
| | BOPF | 0.70 |
| | BOP | 0.65 |
| | BOPM | 0.60 |
| | BPS | 0.55 |
| Fannings/Dust | PF | 0.50 |
| | PD | 0.45 |
| | Dust | 0.50 |

For other made-tea varieties, such as green tea, oolong tea, or white tea, corresponding PGS values can be assigned based on industry standards, sensory evaluation metrics, or user-defined grading parameters. The system allows users to customize PGS values for different tea types, ensuring flexibility in quality level score (QLS) computation across various tea processing techniques.

Now at step 322 of the method 300, the one or more hardware processors (104) is configured to assess the final grade value of the made-tea sample based on the quality level score (QLS) and a valuation grade (VG), where the valuation grade is a quantitative measure of sensory attributes (body and zing) derived from spectral reflectance data and the TDS measurement value, predicted using classification models including Support Vector Machines (SVM), Random Forest (RF), Artificial Neural Networks (ANN), Gradient Boosting Decision Trees (GBDT), k-Nearest Neighbors (k-NN), and other supervised learning classifiers.

The final grade validator 212 validates the final grade value of the made-tea sample. The final grade value is a product of weighting factors, the quality level score (QLS) and the valuation grade (VG), wherein the valuation grade is a sum of body and zing attributes derived from the spectral reflectance data and the TDS measurement value.

The final grade value of the made-tea is defined using Equation 5, $FTI = a \times QLS + b \times VGnorm$

The valuation grade *VGnorm* of the made-tea is defined using Equation 6, $VGnorm = \frac{BodyGrade + ZingGrade}{10}$

Where a and b are weighting factors, ensuring *a* + *b* = 1.

The predefined final grade threshold is categorized as, $HighQuality FTI \geq 0.80 ,$ $MediumQuality : 0.60 \leq FTI < 0.80 ,$ and $LowQuality : FTI < 0.60$

At step 324 of the method 300, the one or more hardware processors (104) are configured to execute factory sample proximity recommendations by generating a quality assessment report indicative of the final grade value, the quality level score, and a proximity value derived from factory-specific reference spectral profiles. The proximity value is determined by computing the degree of alignment between the acquired spectral reflectance profile of the made-tea sample and the predefined spectral reference profiles, wherein spectral deviations are analysed to generate processing recommendations for subsequent tea batches.

Further, the factory recommender 214 includes a proximity value assessor which is configured to perform a machine-learning-based probability score computation by computing spectral profile discrepancies and assessing the probability of the made-tea sample meeting predefined factory-specific standards.

The machine-learning-based probability score computation compares the multi-spectral reflectance data of the made-tea sample against a predefined spectral reference profile to detect wavelength-specific discrepancies. The feature extractor 204 is applied to identify high-impact spectral deviations by analyzing critical wavelength bands associated with key tea chemical compounds, including but not limited to theaflavins, catechins, poly-phenols, and caffeine. Machine learning (ML) models (e.g., Random Forest, Gradient Boosting Decision Trees, Neural Networks) are used to compute a probability score (*Pₛ*) indicative of the likelihood that the made-tea sample conforms to the target spectral profile. The probability score computation follows these steps where the extracted spectral deviation features are computed as shown in Equation 7, $Deviation = {\sum }_{i = 1}^{n} \left(\left|\left({S}_{i}-{T}_{i}\right)\right|\right)$

Feeding the extracted spectral deviations into the ML model, the probability score output (*Pₛ*) is computed based on the magnitude of deviation from the reference spectral profile. The computed probability score (*Pₛ*) is compared against the predefined threshold (*Tp* = 0.90). If *Pₛ* < *Tₚ* an automated process-level correction is triggered to align the spectral profile with the predefined quality standard.

Further, this executes a real-time processing recommendation where processing parameters are dynamically adjusted based on spectral deviations computed in the quality gap analysis. For process modifications, the factory recommender 214 learns continuously from past probability scores and adjusts process-level recommendations using an adaptive ML-based feedback loop. If *Pₛ < Tₚ,* this autonomously generates customized process recommendations for modifying key tea manufacturing parameters to improve spectral consistency and chemical composition balance based on intelligent process level recommendations execution and withering time adjustment.

In another embodiment, the factory recommender 214 analyzes spectral trends influenced by withering duration on moisture retention and enzymatic oxidation. The spectral profile comparison is performed by computing deviations between the spectral reflectance data of the made-tea sample and predefined factory-specific reference profiles at specific wavelength ranges corresponding to key chemical compounds such as theaflavins, catechins, poly-phenols, caffeine and others. If spectral deviations indicate improper oxidation of poly-phenols, dynamically adjusts recommendations based on historical data ensuring spectral correction trends from past batches influence future adjustments. Example: "Increase withering time by 10 minutes to enhance theaflavins content, based on previous batch improvement trends."

Oxidation level modification is evaluated with spectral variations linked to oxidation conditions, influencing the balance of theaflavins and thearubigins. The extracted spectral deviations are processed through a trained machine learning (ML) model (Random Forest, Gradient Boosting) that computes a probability score (*Pₛ*) measuring alignment with factory-specific quality standards. If deviations suggest an oxidation-induced imbalance, the ML model assigns weightage to the most impactful spectral deviations and LLM-based learning fine-tunes process recommendations: "Modify oxidation time by five minutes to balance theaflavins and thearubigins, as per machine-learning-based spectral pattern analysis."

Drying temperature optimization assesses the thermal impact of drying on volatile compound retention and flavor stability. If drying deviations indicate excessive flavor loss, the system predicts the optimal drying correction based on past successful adjustments: "Adjust drying temperature to 60°C to optimize catechin retention, based on best-performing spectral alignment." Spectral deviations are computed at critical wavelength bands, ensuring temperature adjustments are targeted toward optimizing key chemical properties.

Rolling Technique Enhancement assesses spatial irregularities in tea leaf morphology and their correlation with spectral reflectance inconsistencies. The spectral deviations for each sample are compared at multiple reference points in historical data, refining process adjustments iteratively based on previous batches. If spectral variations indicate rolling inconsistencies, the LLM-enhanced learning system refines corrective actions: "Increase rolling pressure by 5% to improve particle uniformity, as per data-driven anomaly detection trends.

The written description describes the subject matter herein to enable any person skilled in the art to make and use the embodiments. By integrating spectral deviation analysis, machine learning-based probability scoring, and adaptive feedback adjustments, the system ensures precise made-tea quality assessment. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of present disclosure herein addresses unresolved problem of made-tea quality assessment. The embodiment, thus provides method and system for made-tea quality assessment. Moreover, the embodiments herein further provides multi-layered anomaly detection framework capable of identifying spectral, visual, and morphological anomalies, thereby enhancing product purity and ensuring compliance with factory-defined quality benchmarks. This adaptive framework ensures continuous learning and process improvement through real-time feedback loops, reducing batch-to-batch variability, minimizing wastage, and enhancing manufacturing efficiency. The method provides advantages with scalable and automated tea quality assessment ensuring standardized quality control, improved consistency, optimized production workflows, and enhanced market competitiveness across large-scale tea production.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an applicationspecific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computerusable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method (300) for assessing made-tea quality, the method comprising:
randomly collecting (302) a physical grade of a made-tea sample from multiple sections of a production batch in a tea factory via one or more hardware processors, wherein the made-tea sample liquor is formulated with a liquor containing water;
acquiring (304) by a spectral camera under controlled illumination via the one or more hardware processors, a spectral image of the made-tea sample indicative of chemical composition, wherein the spectral camera captures the spectral image over a predetermined wavelength range to form a multi-dimensional spectral hypercube;
preprocessing (306) each slice of the acquired spectral image via the one or more hardware processors by,
i. converting each spectral image into grayscale to reduce colour complexity and to improve intensity variations,
ii. applying Gaussian blur on each grayscale of corresponding spectral image to smooth random noise and soften sharp edge, and
iii. performing histogram equalization on each blurred image to obtain improved contrast of distinguishable spectral features;
applying (308) spectral segmentation on the pre-processed spectral image to mark one or more regions of interests corresponding to tea particle via the one or more hardware processors;
creating (310) via the one or more hardware processors, a bounding box on each candidate region among the one or more region of interests;
extracting (312) via the one or more hardware processors, a plurality of features from each candidate region, wherein the plurality of features includes (i) a spectral reflectance data indicative of chemical profile from the bounding box region superimposed on raw spectral hypercube, (ii) a total dissolved solutions (TDS) measurement value obtained from the liquor used to prepare made-tea sample indicative of approximate dissolved compounds, and (iii) a physical grade anomaly detection parameter identifying presence of foreign particles;
detecting (314) from the plurality of features via the one or more hardware processors, a plurality of anomalies comprising a spectral anomaly, a visual anomaly and a physical grade anomaly, wherein the spectral anomaly detects deviations with the spectral reflectance data attributable to heavy metal contamination(s), the visual anomaly detects at least one physical contaminants discrepancies, and the physical grade anomaly validates a plurality of morphological features in comparison with factory provided grade standards;
assigning (316) via the one or more hardware processors, a negative score to each detected anomaly based on severity and type of impact, and recommending corrections for each detected anomaly based on the negative score, wherein the negative score includes a high negative indicating critical discrepancies, a moderate negative indicating moderate discrepancies, and a low negative indicating minor discrepancies;
estimating (318) chemical composition of the made-tea by correlating the spectral reflectance data with the TDS measurement value via the one or more hardware processors;
computing (320) via the one or more hardware processors, a quality level score by combining the chemical composition, the physical grade anomaly and the negative score, and categorizing the quality level score into at least one of a high, medium and low based on a predefined threshold;
assessing (322) via the one or more hardware processors, a final grade value for the made-tea sample based on the quality level score (QLS) and a valuation grade score (VLS), and the final grade value is compared with a predefined grade threshold to quantify factory standards, wherein the valuation grade score is a quantitative measure of body and zing attributes using the spectral reflectance data and the TDS value; and
recommending (324) the tea factory via the one or more hardware processors, a quality assessment report indicating personalized improvements based on the final grade value indicative of acceptable quality, the quality level score, and a proximity value, wherein the proximity value is determined based on factory-specific reference spectral profiles.

2. The processor implemented method as claimed in claim 1, wherein the bounding box on each candidate region is created by,
scanning a masked version of the preprocessed spectral image to identify one or more non-zero pixel clusters that represents valid made-tea region of interests;
randomly selecting each candidate region from the one or more non-zero pixel clusters to minimize systematic bias in bounding box placement; and
creating the bounding box for each candidate region by performing bounding box verification based on at least one of the criteria: (a) each candidate region lies within boundaries of the preprocessed spectral image masks, (b) each candidate region must not overlap with previously defined bounding boxes or non-segmented regions, (c) each candidate region covers localized regions of the made-tea tea particle without enclosing extraneous pixels and (d) each candidate region boundary box regions optimal aspect ratio constraints are constant.

3. The processor-implemented method as claimed in claim 1, wherein the spectral segmentation is applied on the pre-processed spectral image to mark one or more regions of interests by,
applying a clustering technique on the pre-processed spectral image to create a coarse segmentation that differentiates the made-tea sample from the background and outlier pixels;
creating a circular mask around each coarse segmentation of the preprocessed spectral image based on a minimal radius, wherein the minimal radius is determined based on at least one of: a first radius equal to half of the minimal image dimension, excluding peripheral noise, and a second radius based on the edge of the noisy boundary identified after the initial clustering-based segmentation; and
re-executing the spectral segmentation within the circular mask of each coarse segmentation to exclude edges for spectral feature refinement by,
localizing spectral smoothing to reduce pixel noise and to prevent false boundary formation, and
performing adaptive spectral outlier rejection to discard noninformative spectral variations caused by illumination inconsistencies to generate candidate regions corresponding to the made-tea particles for precise bounding box placement.

4. The method as claimed in claim 1, wherein the spectral anomalies are detected by:
continuously monitoring the spectral camera parameters including gain, exposure time, brightness, contrast, and shutter speed and dynamically adjusting the spectral camera parameters to optimize spectral reflectance calibration;
modifying illumination intensity if spectral inconsistencies persist after the spectral camera adjustments; and
comparing the spectral reflectance data of each bounding box with a reference spectral reflectance dataset to quantify deviations and contaminants,
wherein the visual anomaly is detected from the plurality of features to identify physical contaminants discrepancies present in the made tea by,
detecting physical contaminants such as foreign objects within the made-tea samples using image processing techniques; and
comparing multi-spectral data captured at different wavelength intervals to enhance differentiation between organic made-tea structures and non-organic contaminants,
wherein the physical grade anomaly is detected from the processed tea leaves used to prepare the made-tea by,
detecting a plurality of morphological features from each bounding box with a predetermined physical grade benchmark of made-tea; and
applying a pattern recognition technique to validate the plurality of morphological features to flag the tea grade, and an alert is provided when substantial deviations suggest adulteration or mislabeling.

5. The processor implemented method as claimed in claim 1, wherein the high negative score is assigned to spectral anomaly detected if camera related parameters are identified out of tolerance, signifying an elevated risk of misinterpretation of spectral data and the need for immediate recalibration,
wherein the low negative score is assigned to the visual anomalies if physical contaminants reflect lower impact compared to camera and spectral anomalies, and
wherein the moderate negative score is assigned to the physical grade anomaly if harmful metal contaminants are detected which is dynamic and based on the type and concentration of the contaminants.

6. The processor implemented method as claimed in claim 1, wherein the quality level score is computed based on a chemical composition score, a physical grade average score and a weighting factor,
wherein the chemical composition score is a difference between measured concentration of compound and optimal concentration of compound and a total number of compounds, and
wherein the physical grade average score is a product of proportion of physical grade j in the made-tea sample defined between 0 to 1 with a physical grade score for grade j,
wherein the quality level score is categorized as high quality if the value exceeds a first threshold, a medium quality if the quality level score is between a second threshold and the first threshold, and low quality if the quality level score exceeds a third threshold,
wherein the final grade value is a product of weighting factors, the quality level score, and the valuation grade, and
wherein the valuation grade is a sum of body and zing attributes derived from the spectral reflectance data and the TDS measurement value.

7. The processor implemented method as claimed in claim 1, wherein the proximity value is determined by,
performing quality analysis by comparing multispectral data captured at different wavelength with a factory specific reference spectral profiles, wherein each spectral profile represents a target quality outcome under optimal processing conditions;
computing, for the segmented regions of the made-tea sample, a probability score that indicates alignment with each reference spectral profile, from which the proximity value is derived, wherein the probability score is computed based on the magnitude of deviation from the reference spectral profile; and
recommending process level adjustments if the proximity value is below a predefined proximity threshold, wherein the process level adjustments includes at least one of withering time modification, oxidation level tuning, drying temperature optimization, rolling technique alteration for subsequent batches of the tea factory closer to the target profile.

8. A system (100) for assessing made-tea quality, comprising:
memory (102) storing instructions;
one or more communication interfaces (106); and
one or more hardware processors (104) coupled to the memory (102) via the one or more communication interfaces (106), wherein the one or more hardware processors (104) are configured by the instructions to:
randomly collect a physical grade of a made-tea sample from multiple sections of a production batch in a tea factory, wherein the made-tea liquor sample is formulated with a liquor containing water;
acquire by a spectral camera under controlled illumination, a spectral image of the made-tea sample indicative of chemical composition, wherein the spectral camera captures the spectral image over a predetermined wavelength range to form a multi-dimensional spectral hypercube;
preprocess each slice of the acquired spectral image by,
i. converting each spectral image into grayscale to reduce colour complexity and to improve intensity variations,
ii. applying Gaussian blur on each grayscale of corresponding spectral image to smooth random noise and soften sharp edge, and
iii. performing histogram equalization on each blurred image to obtain improved contrast of distinguishable spectral features;
apply spectral segmentation on the pre-processed spectral image to mark one or more regions of interests corresponding to tea particle;
create a bounding box on each candidate region among the one or more region of interests;
extract a plurality of features from each candidate region, wherein the plurality of features includes (i) a spectral reflectance data indicative of chemical profile from the bounding box region superimposed on raw spectral hypercube, (ii) a total dissolved solutions (TDS) measurement value obtained from the liquor used to prepare made-tea sample indicative of approximate dissolved compounds, and (iii) a physical grade anomaly detection parameter identifying presence of foreign particles;
detect from the plurality of features a plurality of anomalies comprising a spectral anomaly, a visual anomaly and a physical grade anomaly, wherein the spectral anomaly detects deviations with the spectral reflectance data attributable to heavy metal contamination(s), the visual anomaly detects at least one physical contaminants discrepancies, and the physical grade anomaly validates a plurality of morphological features in comparison with factory provided grade standards;
assign a negative score to each detected anomaly based on severity and type of impact, and recommending corrections for each detected anomaly based on the negative score, wherein the negative score includes a high negative indicating critical discrepancies, a moderate negative indicating moderate discrepancies, and a low negative indicating minor discrepancies;
estimate a chemical composition of the made-tea by correlating the spectral reflectance data with the TDS measurement value;
compute a quality level score by combining the chemical composition, the physical grade anomaly and the negative score, and categorizing the quality level score into at least one of a high, medium and low based on a predefined threshold;
assess a final grade value for the made-tea sample based on the quality level score (QLS) and a valuation grade score (VLS), and the final grade value is compared with a predefined grade threshold to quantify factory standards, wherein the valuation grade score is a quantitative measure of body and zing attributes using the spectral reflectance data and the TDS value; and
recommend the tea factory, a quality assessment report indicating personalized improvements based on the final grade value indicative of acceptable quality, the quality level score, and a proximity value, wherein the proximity value is determined based on factory-specific reference spectral profiles.

9. The system as claimed in claim 8, wherein the bounding box on each candidate region is created by,
scanning a masked version of the preprocessed spectral image to identify one or more non-zero pixel clusters that represents valid made-tea region of interests;
randomly selecting each candidate region from the one or more non-zero pixel clusters to minimize systematic bias in bounding box placement; and
creating the bounding box for each candidate region by performing bounding box verification based on at least one of the criteria: (a) each candidate region lies within boundaries of the preprocessed spectral image masks, (b) each candidate region must not overlap with previously defined bounding boxes or non-segmented regions, (c) each candidate region covers localized regions of the made-tea tea particle without enclosing extraneous pixels and (d) each candidate region boundary box regions optimal aspect ratio constraints are constant.

10. The system as claimed in claim 8, wherein the spectral segmentation is applied on the pre-processed spectral image to mark one or more regions of interests by,
applying a clustering technique on the pre-processed spectral image to create a coarse segmentation that differentiates the made-tea sample from the background and outlier pixels;
creating a circular mask around each coarse segmentation of the preprocessed spectral image based on a minimal radius, wherein the minimal radius is determined based on at least one of: a first radius equal to half of the minimal image dimension, excluding peripheral noise, and a second radius based on the edge of the noisy boundary identified after the initial clustering-based segmentation; and
re-executing the spectral segmentation within the circular mask of each coarse segmentation to exclude edges for spectral feature refinement by,
localizing spectral smoothing to reduce pixel noise and to prevent false boundary formation, and
performing adaptive spectral outlier rejection to discard noninformative spectral variations caused by illumination inconsistencies to generate candidate regions corresponding to the made-tea particles for precise bounding box placement.

11. The system as claimed in claim 8, wherein the spectral anomalies are detected by:
continuously monitoring the spectral camera parameters including gain, exposure time, brightness, contrast, and shutter speed and dynamically adjusting the spectral camera parameters to optimize spectral reflectance calibration;
modifying illumination intensity if spectral inconsistencies persist after the spectral camera adjustments; and
comparing the spectral reflectance data of each bounding box with a reference spectral reflectance dataset to quantify deviations and contaminants,
wherein the visual anomaly is detected from the plurality of features to identify physical contaminants discrepancies present in the made tea by,
detecting physical contaminants such as foreign objects within the made-tea samples using image processing techniques; and
comparing multi-spectral data captured at different wavelength intervals to enhance differentiation between organic made-tea
structures and non-organic contaminants,
wherein the physical grade anomaly is detected from the processed tea leaves used to prepare the made-tea by,
detecting a plurality of morphological features from each bounding box with a predetermined physical grade benchmark of made-tea; and
applying a pattern recognition technique to validate the plurality of morphological features to flag the tea grade, and an alert is provided when substantial deviations suggest adulteration or mislabeling.

12. The system as claimed in claim 8, wherein the high negative score is assigned to spectral anomaly detected if camera related parameters are identified out of tolerance, signifying an elevated risk of misinterpretation of spectral data and the need for immediate recalibration,
wherein the low negative score is assigned to the visual anomalies if physical contaminants reflect lower impact compared to camera and spectral anomalies, and
wherein the moderate negative score is assigned to the physical grade anomaly if harmful metal contaminants are detected which is dynamic and based on the type and concentration of the contaminants.

13. The system as claimed in claim 8, wherein the quality level score is computed based on a chemical composition score, a physical grade average score and a weighting factor,
wherein the chemical composition score is a difference between measured concentration of compound and optimal concentration of compound and a total number of compounds,
wherein the physical grade average score is a product of proportion of physical grade j in the made-tea sample defined between 0 to 1 with a physical grade score for grade j,
wherein the quality level score is categorized as high quality if the value exceeds a first threshold, a medium quality if the quality level score is between a second threshold and the first threshold, and low quality if the quality level score exceeds a third threshold,
wherein the final grade value is a product of weighting factors, the quality level score, and the valuation grade, and
wherein the valuation grade is a sum of body and zing attributes derived from the spectral reflectance data and the TDS measurement value.

14. The system as claimed in claim 8, wherein the proximity value is determined by,
performing quality analysis by comparing multi-spectral data captured at different wavelength with a factory-specific reference spectral profiles, wherein each spectral profile represents a target quality outcome under optimal processing conditions;
computing for the segmented regions of the made-tea sample, a probability score that indicates alignment with each reference spectral profile, from which the proximity value is derived, wherein the probability score is computed based on the magnitude of deviation from the reference spectral profile; and
recommending process-level adjustments if the proximity value is below a predefined proximity threshold, wherein the process-level adjustments includes at least one of withering time modification, oxidation level tuning, drying temperature optimization, rolling technique alteration for subsequent batches of the tea factory closer to the target profile.

15. One or more non-transitory machine-readable information storage mediums comprising one or more instructions which when executed by one or more hardware processors cause:
randomly collecting a physical grade of a made-tea sample from multiple sections of a production batch in a tea factory, wherein the made-tea sample liquor is formulated with a liquor containing water;
acquiring by a spectral camera under controlled illumination, a spectral image of the made-tea sample indicative of chemical composition, wherein the spectral camera captures the spectral image over a predetermined wavelength range to form a multi-dimensional spectral hypercube;
preprocessing each slice of the acquired spectral image by,
i. converting each spectral image into grayscale to reduce colour complexity and to improve intensity variations,
ii. applying Gaussian blur on each grayscale of corresponding spectral image to smooth random noise and soften sharp edge, and
iii. performing histogram equalization on each blurred image to obtain improved contrast of distinguishable spectral features;
applying spectral segmentation on the pre-processed spectral image to mark one or more regions of interests corresponding to tea particle;
creating, a bounding box on each candidate region among the one or more region of interests;
extracting, a plurality of features from each candidate region, wherein the plurality of features includes (i) a spectral reflectance data indicative of chemical profile from the bounding box region superimposed on raw spectral hypercube, (ii) a total dissolved solutions (TDS) measurement value obtained from the liquor used to prepare made-tea sample indicative of approximate dissolved compounds, and (iii) a physical grade anomaly detection parameter identifying presence of foreign particles;
detecting from the plurality of features, a plurality of anomalies comprising a spectral anomaly, a visual anomaly and a physical grade anomaly, wherein the spectral anomaly detects deviations with the spectral reflectance data attributable to heavy metal contamination(s), the visual anomaly detects at least one physical contaminants discrepancies, and the physical grade anomaly validates a plurality of morphological features in comparison with factory provided grade standards;
assigning, a negative score to each detected anomaly based on severity and type of impact, and recommending corrections for each detected anomaly based on the negative score, wherein the negative score includes a high negative indicating critical discrepancies, a moderate negative indicating moderate discrepancies, and a low negative indicating minor discrepancies;
estimating chemical composition of the made-tea by correlating the spectral reflectance data with the TDS measurement value;
computing, a quality level score by combining the chemical composition, the physical grade anomaly and the negative score, and categorizing the quality level score into at least one of a high, medium and low based on a predefined threshold;
assessing, a final grade value for the made-tea sample based on the quality level score (QLS) and a valuation grade score (VLS), and the final grade value is compared with a predefined grade threshold to quantify factory standards, wherein the valuation grade score is a quantitative measure of body and zing attributes using the spectral reflectance data and the TDS value; and
recommending the tea factory, a quality assessment report indicating personalized improvements based on the final grade value indicative of acceptable quality, the quality level score, and a proximity value, wherein the proximity value is determined based on factory-specific reference spectral profiles.
